# EUROPEAN PATENT SPECIFICATION

(11) **EP 1 410 245 B1**
(45) Date of publication and mention of the grant of the patent: **12.10.2005**
(21) Application number: 01270618.0
(22) Date of filing: 14.12.2001
(51) Int. Cl.: G06F 17/30

(54) **FOCUSSING OF COMPOUND LIBRARIES USING ATOMIC ELECTROTOPOLOGICAL VALUES**
Fokussierung von Komponentendatenbanken anhand von atomischen elektrotopologischen Werten
CONCENTRATION DE BIBLIOTHEQUES COMPOSITES AU MOYEN DE VALEURS ATOMIQUES ELECTROTOPOLOGIQUES

(30) Priority: 15.12.2000 US 255449 P
(43) Date of publication of application: 21.04.2004
(73) Proprietor: The Genetics Company Inc., 8952 Schlieren (CH)
(72) Inventor: ENGKVIST, Ola, 16761 Hennigsdorf (DE); WREDE, Paul, 14195 Berlin (DE)
(74) Representative: Dey, Michael
(86) International application number: PCT/EP2001/014807
(87) International publication number: WO 2002/048389

(56) References cited:
- OSMAN F. GÜNER: "Pharmacophore Perception, Development, and Use in Drug Design" 26 July 2000 (2000-07-26) , INTERNATIONAL UNIVERSITY LINE , LA JOLLA, CA, USA XP002257096 page 70 -page 77
- L. B. KIER, L. H. HALL: "Molecular Structure Description. The Electrotopological State" 1999 , ACADEMIC PRESS , SAN DIEGO, CA, USA XP002257097 cited in the application page 62 -page 65 page 123 -page 129
- BÖHM, KLEBE, KUBINYI: "Wirkstoffdesign. Der Weg zum Arzneimittel" , SPEKTRUM, AKADEMISCHER VERLAG , HEIDELBERG XP002257098 cited in the application page 381 -page 397 figure 21.5 figure 21.6
- PICKETT S D ET AL: "DIVERSITY PROFILING AND DESIGN USING 3D PHARMACOPHORES: PHARMACOPHORE-DERIVED QUERIES (PDQ)" JOURNAL OF CHEMICAL INFORMATION AND COMPUTER SCIENCES, AMERICAN CHEMICAL SOCIETY, COLOMBUS,OHIO, US, vol. 36, 1996, pages 1214-1223, XP002062096 ISSN: 0095-2338 cited in the application

## Description

The present invention relates to a method for generating a focussed compound library containing an enriched amount of ligand compounds being capable of binding to a predetermined receptor. The focussing of the library can be performed according to predetermined biological activities or properties of the compounds.

### Background of the invention

The search and evaluation of new drugs or drug targets on short time scales requires the use of high-throughput screening (HTS) in pharmaceutical research. Beside the screening of real compounds, it is also possible to determine new drug targets using computational screening methods. The application of computational screening is often called virtual screening.

The bottleneck of current drug discovery and drug development is the large screening demand of pharmaceutically relevant targets. To circumvent this difficulty a lot of effort is put on improvement of effective virtual (computer) screening tools. Currently combinatorial chemistry and high-throughput screening is effective in the search of new lead structures. However, these processes are still very expensive and time-consuming. Therefore, computer-based algorithms can be a significant improvement in minimizing costs and time.

In the current screening processes a hit rate of about 0.1% can be reached. Thus, an increase of the hit rate is desirable, which will improve the screening process and reduce costs and time.

In "Pharmacophore Perception ,Development and Use in Drug Design", Osman F. Güner, Chapter 5 "HipHop: Pharmacophores Based on Multiple Common-Feature Alignments", pp. 70-77, O.O. Clement, A. T. Mehl, a methodology employed in automated pharmacophore model generation using the Catalyst/HipHop program is described.

Tools that can screen databases for molecules with biological activity are of tremendous value for the pharmaceutical and biotechnological industry. A common scenario is that one or several molecules showing biological activity for a specific target is known. This/These molecule(s) are used to screen a database of molecules for other molecules with similar activity. Methods that have been developed in this area, for instance, are CATS (Schneider et al., Angewandte Chemie int.ed. 1999, 38, 2894-2896), CATALYST (Barnum et al., J.Chem.Inf.Comput.Sci. 1996, 36, 563-571) and PHACIR (U.S. Application No.09/634,586). These methods have three essential parts: 1. Description of the molecular geometry. 2. Translation of the atomic properties of the molecule into a pharmacophore model. 3. Comparisons of the pharmacophores for different molecules.

So far many methods are based on a search of individual chemical structures as new drug-lead compounds. The information used is derived either from existing active compounds or from the target structure. These conventional methods consider each structure separately and search for similarities with the known active compound or for complementarities to a protein-target binding site. However, these methods can be applied only to databases having a limited number of members, since the algorithms used are rather time consuming. However, an increasing number of potential drug candidates can be synthesized, e.g. by combinatorial chemistry or can be virtually generated. It is desirable to provide methods, allowing fast screening of large databases or reducing the total number of members of databases while at the same time increasing the percentage of hits within the database.

It is therefore an objective of the present invention to provide a method to further improve and accelerate the virtual screening of databases for specific ligand compounds.

It is a further objective of the present invention to provide a method which allows for generating a focussed compound library containing an enriched amount of ligand compounds being capable of binding to a predetermined receptor.

### Summary of the invention

The present invention is related to a novel ligand detection system. The method according to the invention identifies ligands, which are capable of specific binding to a given receptor or to given protein domains. An algorithm, preferably computer-based, can generate a focussed compound library which enriches the active compounds. Screening of such an enriched library results in a significant increase of the hit rate. The basic concept underlying the applied algorithms is a new way of translating the atomic information of a molecule into a pharmacophore model.

The normal way of translating information about the properties of a molecule into pharmacophores is to assign atoms as different atomic types, traditionally atoms have been described as hydrogen bond donors, hydrogen bond acceptors, lipophilic, aromatic, positive or negative. In general, a pharmacophore describes the three-dimensional order of all atoms within a ligand which can interact with a receptor (Böhm, H.-J., Klebe, G., Kubinyi, H.; Wirkstoffdesign, Spektrum Verlag, Heidelberg, 1996). The pharmacophore concept, suitable interaction types and distance ranges are described e.g. by S.D. Pickett et al., J. Chem. Inf. Comput. Sci. 36 (1996), 1214-1232; H.-J. Böhm, G. Klebe, H. Kubinyi, Wirkstoffdesign, Spektrum Verlag, Heidelberg, 1996; S.M. Brocklehurst et al., Creating Integrated Computer Systems for Target Discovery and Drug Discovery, Pharmainformatics Elsevier Science Ltd., (1999), pages 12-15 and J.S. Mason, Computational Screening: Large-scale Drug Discovery, Pharmainformatics Elservier Science Ltd., (1999), pages 34-36.

The invention particularly includes the enrichment of biologically active compounds in a focussed substance library, which is obtained from given starting databases. First the relevant biological properties are extracted from compounds with known biological activity and described in the form of pharmacophores. Pharmacophores produced this way can then be used for the extraction of similar compounds from a substance library or for focussing a database according to specific similarity criteria. It is very advantageous that the use of pharmacophores enables very rapid screening also of large databases. During the screening process a focussed substance library is generated, which is smaller than the originally used starting database and which contains an enormously increased proportion of active substances compared to the original substance library.

An essential feature of the method according to the invention is the generation of pharmacophores based on atomic types obtained using atomic electrotopological (AET) values. According to the method of the invention, which is also named PHATS, pharmacophores are evaluated in a completely different way than in the state of the art. Atomic electrotopological (AET) values are calculated for each atom and used as atomic types. AET values are usually between -2 and 12. Atoms with AET values between two specified boundaries are assigned to belong to the same atomic type. The method of calculating AET values has been developed by Kier & Hall (Molecular Structure Description, The Electrotopological State, Academic Press, London, 2000). However, AET values have never been used in conjunction with the molecular geometry to screen for biologically active molecules. The atomic types are then used to construct a pharmacophore model. The new way of describing the atomic types has several advantages compared to the old one. Atomic types are assigned automatically by the computer, there is no need for a person to predefine what is a hydrogen bond donor, a lipophilic atom and so on. Most important, since AET values are assigned by the computer, the number of atomic types and the boundary values between different atomic types can be optimized for each specific target. The values can be optimized for a small test library and can then be used to screen large databases to find new lead compounds. This means that a specific pharmacophoric model can be developed for each target type instead of using the same for each target as in earlier models. A specifically designed pharmacophore model will screen a database more efficiently for active molecules of that specific target type. Calculations have shown that this new way of creating a pharmacophore model is superior to the traditional one. Further, with the method according to the invention biologically relevant interactions can be considered.

The method according to the invention for the generation of a focussed substance library generally comprises the following steps:
1. The molecules (i.e. the known ligand(s) and compound(s) of a starting database) are described with a code in computer-readable form, e.g. the SMILES code (D.Weininger, J.Chem.Inf.Comput.Sci. 28 (1988), 31-36).
2. The geometry of the molecules is described either with their bond distance matrix or with their three-dimensional coordinates.
3. Atomic electrotopological indexes are evaluated for each atom, the values are used to assign the atoms as different atomic types.
4. The molecule is described with pharmacophores, e.g. two-, three-, or four-point pharmacophores, using these atomic types.
5. The collection of the pharmacophores is compared using a similarity index, e.g. with the distance between two vectors or with the Tanimoto coefficient.

For the preparation of the pharmacophores particularly used are: one or more compounds having known biological activity, one or more compounds each having a known active three-dimensional conformation, one or more compounds, whose intermolecular interactions are derived from known interactions of ligand-protein complexes together with the receptor or one or more receptors with known three-dimensional structures for each a given biologically relevant property, e.g. the binding affinity to a receptor.

The method according to the invention can be used for the virtual screening of substance libraries and can be applied in particular for the development of drugs or biologically active compounds. It is particularly suitable for applications in human or veterinary medicine and in plant protection. Examples for indications are oncology, cardio-vascular diseases, neurology, metabolic diseases, infectious diseases and virology. The method can also be applied in searches of substances, which shall be used for the modulation or inhibition of receptor-ligand interactions. Thus, the invention particularly can be applied to find new lead structures for pharmaceutical, biotechnological and agrochemical targets.

The method according to the invention allows to convert a database of arbitrary size into a focussed substance library of much smaller size, the members of which can be further evaluated, e.g in experimental tests. With the method according to the invention a database is sorted according to other substances capable of binding, wherein it was detected that more than 40% and in particular 60-80% of the actual hits are placed in the focussed substance library. When the substances of a database are sorted using the inventive method, a substance library of arbitrary size can be generated, e.g. by selecting the 10% best hits, the 20% best hits or the 50% best hits. Preferably a reduction of the starting database to less than 1/3 of its original size, more preferably to less than 1/5 of its original size is carried out. Due to a limiting pre-selection, e.g. using the criterium 1 % best hits or just the very best hit, it is further possible to synthesize also complicated structures from this focussed substance library, optionally in a multi-step process and subsequently investigate them experimentally. This synthesizing step requires only little effort, compared to conventional approaches taking into account the large amount of molecules contained in the original database and the large amount of molecules having little or no binding affinity existing therein.

The described procedures of virtual screening are unique and innovative and result in an efficient sorting of databases, which allows a significant enrichment for biologically active molecules even within very large databases. The method of the invention preferably provides an enhancement of the proportion of molecules having the desired activity or properties. The enhancement can be described as enrichment factor (EF), which is defined as: EF = δ (focussed library) / δ (whole library) with the density δ = number of active compounds / total number of compounds. The enrichment factor El is preferably greater than 2, more preferably greater than 3.

The virtual screening methods can be applied to three point pharmacophores and can easily be extended to four point pharmacophores to be able to consider the chirality of the molecules, which play an additional role for binding specifity.

The term "pharmacophore" as used herein refers to the sum of all ligand atoms which have intermolecular interactions to the receptor.

The invention can be summarized as follows:

The invention, in general, relates to the identification of biologically active molecules with virtual screening, using the following steps:
- Description of the molecules as a computer-readable code, e.g. a SMILES string.
- Description of the molecule e.g. with its three-dimensional geometry or with a bond distance matrix. This information can be extracted from the SMILES code.
- Description of atomic types based on the atomic electrotopological values. The AET values are calculated for each atom and are usually between -2 and 12. The AET value is specific for each atom depending on the environment/surroundings of the atom. Usually 3-5 different atomic typs are defined within the invention. The grouping of AET values into atomic types can be performed, for example, as follows: Atomic type | Δ AET values from -∞ to < 0; atomic type II Δ AET values from 0 to < 5; atomic type III Δ AET values from 5 to < 10 and atomic type IV Δ AET values from 10 to + ∞. However, other groupings are also possible and might prove advantageous for specific target types.
- The pharmacophore is based on these atomic types.
- Construction and enumeration of pharmacophores, e.g. of all possible two-, three- or 4-point pharmacophores.
- Optionally, the specific atomic electrotopological values that are included in an atomic type and the total number of atomic types can be optimized by screening a test libary containing known binding (hits) and non-binding structures.
- The optimized atomic types can be used to construct pharmacophores. The optimized pharmacophore models then are used to screen large databases for active molecules.
- With the method according to the invention one can sort a molecular database according to the molecules similarity and/or according to biological activity.
- The method according to the invention can be used to find new lead structures for pharmaceutical, biotechnological and agrochemical targets.

### Detailed description of the invention

The invention can be applied for generating a focussed library either when only ligands are known, when the three-dimensional structure of a receptor-ligand complex is known or when the sole receptor structure is available.

Thus, the invention relates to a method for generating a focussed compound library from a starting compound library wherein said focussed compound library contains an enriched amount of ligand compounds being capable of binding to a predetermined receptor, comprising the steps:
(a) providing at least one structure of a ligand, a ligand-receptor complex or a ligand binding site geometry for the predetermined receptor,
(b) generating a computer-readable code of said at least one structure,
(c) providing a description of said at least one structure in the form of its three-dimensional geometry or/and of its bond distance matrix,
(d) providing atomic eletrotopological values for the atoms of said at least one structure;
(e) generating atomic types based on said atomic electrotopological values,
(f) generating pharmacophores based on said atomic types,
(g) sorting a starting database with said pharmacophores, using a similarity index by
   (g1) providing a description of the structure of the compounds contained in the database in the form of their three-dimensional geometry or/and of their bond distance matrix,
   (g2) providing atomic electrotopological values for the atoms of said at least one structure,
   (g3) generating atomic types based on said atomic electrotopological values,
   (g4) generating pharmacophores based on said atomic types, and
   (g5) comparing the pharmacophores of said at least one ligand structure with the pharmacophores of the database compounds,
(h) determining a ranking of the database compounds according to the detected similarities, and
(i) obtaining a focussed compound libary having an enriched amount of ligand compounds.

In a first preferred embodiment the invention comprises a method for generating a focussed compound library from a starting compound library wherein said focussed compound library contains an enriched amount of ligand compounds being capable of binding to a predetermined receptor, comprising the steps:
(a) providing at least one ligand structure for the predetermined receptor,
(b) generating a predetermined number of possible ligand conformers of said ligand structure,
(c) generating a computer-readable code of the possible ligand conformers,
(d) providing a description of the possible ligand conformers in the form of their three-dimensional geometry or/and of their bond distance matrix,
(e) providing atomic eletrotopological values for the atoms of the possible ligand conformers,
(f) generating atomic types based on said atomic eletrotopological values,
(g) generating pharmacophores based on said atomic types,
(h) sorting a starting database with said pharmacophores using a similarity index by
   (h1) generating a predetermined number of conformers for compounds contained in the database,
   (h2) providing a description of the structure of said conformers in the form of their three-dimensional geometry or/and their bond distance matrix,
   (h3) providing atomic eletrotopological values for the atoms of said conformers,
   (h4) generating atomic types based on said atomic electrotopological values,
   (h5) generating pharmacophores for said conformers of said database compounds based on said atomic types and
   (h6) comparing the pharmacophores of the ligand structure with the pharmacophores of the database compounds and
(i) determining a ranking of the database compounds according to the detected similarities,
(j) obtaining a focussed compound library having an enriched amount of ligand compounds.

The starting point in this approach is that one or more ligands for a predetermined receptor, for which possible ligand compounds are to be searched for, is known. A given compound library is screened by the information obtained from the known ligand structure(s). In a first step a ligand structure for the predetermined receptor is provided. Next, optionally a certain predetermined number of possible ligand conformers of said known ligand structure is generated. Preferably, the predetermined number (m) of generated conformers for the known ligand structure is at least 1, more preferably at least 5 and most preferably at least 10. Theoretically, the upper value of the predetermined number of possible ligand conformers is not limited and preferably is less than 1.000, more preferably less than 100 and most preferably less than 20. The conformers can be generated by computational methods, such as force-field, rule-based, semiempiric, or ab initio methods. However, it is also possible to take experimentally determined conformer structures of the known ligand(s).

In a next step a pharmacophore for each of the possible ligand conformers is generated. The pharmocophores are described by the atomic types generated from the AET values for each atom. Preferably, the pharmacophores are based on all possible ligand atoms that could have intermolecular interactions to the receptor and distances.

According to the invention it is preferred to use three-point pharmacophores. However, it is also possible to use two-point or four-point pharmacophores or even higher point pharmacophores.

According to the invention said ligand pharmacophores and preferably all found ligand pharmacophores can be combined to a fingerprint (FP). This fingerprint is an abstract binary representation of the pharmacophores or a common pharmacophore, respectively. The fingerprint concept is described e.g. in Vincent J. van Geerestein, Hans Hamersma and Steven P. van Helden: Exploiting Molecular Diversity: Pharmacophore Searching and Compound Clustering, in: Han van de Waterbeemd et al., Computer-Assisted Lead Finding and Optimization, Verlag Helvetica Chimica Acta Basel (1997), pages 159-178. The use of one fingerprint for screening instead of a large number of pharmacophores greatly reduces the effort necessary for screening large amounts of compounds in a database. Within the fingerprint the characteristic features of the known structures are represented in a simple computer-readable binary form facilitating the comparison of the known ligand structure with possible ligand candidates.

The pharmacophores generated and/or said fingerprint can then be used for a similarity search. Thereby a database can be sorted with the pharmacophores or/and the active fingerprint as a query. For this, a starting database, such as a database of known individual compounds, a database of synthesized combinatorial libraries and/or a database of virtual combinatorial libraries is first converted into a database represented by pharmacophores or/and fingerprints. For this purpose in a first step a predetermined maximum number (m) of conformers for all compounds of the database is generated. Preferably, the predetermined number is at least 1, more preferably at least 5 and most preferably at least 10. While the maximum for the predetermined number is theoretically not limited, it is preferred to generated not more than 1.000, preferably not more than 100, more preferably not more than 50 and most preferably not more than 20 conformers for each compound of the database.

Next, a potential pharmacophore is determined for said conformers of each database compound in the same way as described above with respect of the generation of a pharmacophore for the known ligand structure. If a fingerprint database is desired, fingerprints are generated for the pharmacophores of each conformer of the database compounds and preferably for all pharmacophores of each conformer of the database compounds. The pharmacophores or/and the active fingerprint of the query molecule is then compared with the pharmacophores or/and the fingerprints of the compounds of the library.

A similarity index, such as the Tanimoto coefficent, the Eucledian distance or the Manhattan distance is used to sort all library compounds. Suitable similarity indices are described e.g. in Peter Willet, J.M. Barnard, G.M. Downs: Chemical Similarity Searching, J. Chem. Inf. Comput. Sci. 38 (1998), pages 983-996. During this step a ranking of the database compounds according to the detected similarities is performed and the order of the compounds can be fixed according to their similarity to the known ligand structure.

It is also possible to use vectors for the similarity comparison. The principle of using vectors is explained in the following for an example comprising a model with three atomic types (derived from the AET values) and bond distances between 1 and 10 and a two-point pharmacophore model. From three atomic types six different two-point pharmacophores can be constructed, and starting with distances up to 10 bond lengths a 60 (6*10) dimensional vector is obtained. Each dimension of the vector describes a specific bond distance between two atoms and the atom types of the two atoms. All possible two-atom combinations in a molecule are calculated and one is added to the dimension that corresponds to each two atom combination. All combinations are summed giving a vector that describes the molecule. Since different molecules have different structure and atomic types, the summed vectors for the two molecules will be different and the Euclidian distance can be calculated between those two vectors. If the distance between the vectors is short (in particular below a predetermined limit), the molecules are considered to be similar to each other. This means that several compounds of a library can be ranked with respect to their similarity to one (or several) active molecule(s).

A focussed compound library with a reduced number of total members compared to the original database can be obtained by simply selecting a specific number, such as e.g. 1, 10, 100, 1.000 or 2.000 or a specific percentage, e.g. 10% or 20% of the compounds according to their ranking.

It was found that a considerable amount of hits, such as at least 30%, more preferably at least 50% and typically between 60 and 80% are contained in such a focussed compound library giving a significant enrichment with respect to the biologically active molecules.

If more than one ligand with the same function is available, the information of all ligand structures can be used. When two or more ligand structures for the predetermined receptor are known, for each of the N ligands (wherein N is the number of known ligand structures) a certain maximum number (m) of conformers can be generated, e.g. by calculation with an appropriate method as described above. Next, for each conformer the pharmacophores are generated as described above. The pharmacophores then can be combined to a common pharmacophore which is used to query a starting database. Alternatively, a fingerprint for each ligand conformer can be created. These fingerprints are combined with logical operations to a common fingerprint, representing a group of ligands for the same binding site. A database can be sorted with this common fingerprint in the same way as described above with respect to sorting a database with a pharmacophore.

The method of the invention also can be applied, if the three-dimensional structure of a ligand-receptor complex for the predetermined receptor is known. From the three-dimensional structure of the ligand-receptor complex the ligand structure can be derived and the informations for the intermolecular interactions between ligand and receptor are used to build up the pharmacophore model. The generation of conformers is not necessary in this case, since a correct structure of the ligand is already known and fixed by the arrangement within the ligand-receptor complex. The three-dimensional structure can be obtained from experimental data, such as X-ray diffraction, NMR, or they can be obtained by calculation methods. Preferably, the three-dimensional structure is derived from experimental results.

If more than one three-dimensional ligand-receptor-complex structures are known, the ligand structures are derived from the three-dimensional ligand-receptor-complex structures. The pharmacophores of the ligand structures can be generated based on the intermolecular interactions between ligand and receptor, calculating AET values for the atoms involved, while it is not necessary to create possible conformers. The information of the multiple three-dimensional known ligand-receptor-complex structures can be used by combining at least two and preferably all individual pharmacophores or fingerprints to a common pharmacophore or fingerprint, respectively. This common pharmacophore or fingerprint is then used to sort a given database as described above. Alternatively, information of the multiple three-dimensional known ligand-receptor-complex structures can be used by building a common pharmacophore out of at least two and preferably all individual pharmacophores, to combine the information of the different structures. A common fingerprint can then be derived from the common pharmacophore of at least two and preferably of all known three-dimensional structures.

In a further embodiment a pharmacophore model can be derived from a known binding site geometry of the predetermined receptor. In this approach the binding site geometry of said receptor is inverted to create pharmacophores. With these pharmacophores a given database can be sorted as described above.

The invention also comprises systems, apparatuses, disks, computers or other hardware which are adapted or set up to carry out or control one of the methods of the invention.

## Claims

1. A method for generating a focussed compound library from a starting compound library wherein said focussed compound library contains an enriched amount of ligand compounds being capable of binding to a predetermined receptor, comprising the steps:
(a) providing at least one structure of a ligand, a ligand-receptor complex or a ligand binding site geometry for the predetermined receptor,
(b) generating a computer-readable code of said at least one structure,
(c) providing a description of said at least one structure in the form of its three-dimensional geometry or/and of its bond distance matrix,
(d) providing atomic electrotopological values for the atoms of said at least one structure;
(e) generating atomic types based on said atomic electrotopological values, wherein atoms with atomic electrotopological values between two specified boundaries are assigned to the same atomic type,
(f) generating pharmacophores based on said atomic types,
(g) sorting a starting database using said pharmacophores, using a similarity index by
(g1) providing a description of the structure of the compounds contained in the database in the form of their three-dimensional geometry or/and of their bond distance matrix,
(g2) providing atomic electrotopological values for the atoms of said at least one structure,
(g3) generating atomic types based on said atomic electrotopological values,
(g4) generating pharmacophores based on said atomic types, and
(g5) comparing the pharmacophores of said at least one ligand structure with the pharmacophores of the database compounds,
(h) determining a ranking of the database compounds according to the detected similarities, and
(i) obtaining a focussed compound libary having an enriched amount of ligand compounds.

2. A method according to claim 1 for generating a focussed compound library from a starting compound library wherein said focussed compound library contains an enriched amount of ligand compounds being capable of binding to a predetermined receptor, comprising the steps:
(a) providing at least one ligand structure for the predetermined receptor,
(b) generating a predetermined number of possible ligand conformers of said ligand structure,
(c) generating a computer-readable code of the possible ligand conformers,
(d) providing a description of the possible ligand conformers in the form of their three-dimensional geometry or/and of their bond distance matrix,
(e) providing atomic electrotopological values for the atoms of the possible ligand conformers,
(f) generating atomic types based on said atomic electrotopological values, wherein atoms with atomic electrotopological values between two specified boundaries are assigned to the same atomic type,
(g) generating pharmacophores based on said atomic types,
(h) sorting a starting database using said pharmacophores using a similarity index by
(h1) generating a predetermined number of conformers for compounds contained in the database,
(h2) providing a description of the structure of said conformers in the form of their three-dimensional geometry or/and their bond distance matrix,
(h3) providing atomic electrotopological values for the atoms of said conformers,
(h4) generating atomic types based on said atomic electrotopological values,
(h5) generating pharmacophores for said conformers of said database compounds based on said atomic types and
(h6) comparing the pharmacophores of the ligand structure with the pharmacophores of the database compounds and
(i) determining a ranking of the database compounds according to the detected similarities,
(j) obtaining a focussed compound library having an enriched amount of ligand compounds.

3. The method according to claim 1, wherein the similarity index used for sorting the database is selected from the group consisting of Tanimoto coefficient, Eucledian distance, Manhattan distance and any combination thereof.

4. The method according to claim 1, wherein two-point pharmacophores (2PP), three-point pharmacophores (3PP) and/or four-point pharmacophores (4PP) are generated.

5. The method according to claim 2, wherein the conformers are generated by force field or rule based methods or combinations thereof.

6. The method according to claim 2, wherein the predetermined number of conformers is a number between 5 and 20.

7. The method according to claim 1, wherein the starting compound library is selected from a database of known individual compounds, a database of synthesized combinatorial libraries and/or a database of virtual combinatorial libraries.

8. The method according to claim 1, wherein specific atomic electrotopological values that are included in an atomic type and/or the total number of atomic types are optimized by screening a test library.

9. A method according to claim 1 for generating a focussed compound library from a starting compound library wherein said focussed compound library contains an enriched amount of ligand compounds being capable of binding to a predetermined receptor, comprising the steps:
(a) providing a three-dimensional ligand-receptor-complex structure for the predetermined receptor,
(b) deriving the ligand structure from the three-dimensional ligand-receptor-complex structure,
(c) generating a computer readable code of said ligand structure,
(d) providing a description of said ligand structure in the form of its three-dimensional geometry or/and of its bond distance matrix,
(e) providing atomic electrotopological values for the atoms of said ligand structure,
(f) generating atomic types based on said atomic electrotopological values, wherein atoms with atomic electrotopological values between two specified boundaries are assigned to the same atomic type,
(g) generating pharmacophores of the ligand structure based on said atomic types and intermolecular interactions between ligand and receptor,
(h) sorting a starting database using said pharmacophores, using a similarity index by
(h1) generating a predetermined number of conformers for compounds contained in the database,
(h2) determining pharmacophores for said conformers of said database compounds based on atomic types generated using atomic electrotopological values, and
(h3) comparing the pharmacophores of the ligand structure with the pharmacophores of the database compound and
(i) determining a ranking of the database compounds according to the detected similarities and
(j) obtaining a focussed compound library having an enriched amount of ligand compounds.

10. A method according to claim 1 for generating a focussed compound library from a starting compound library wherein said focussed compound library contains an enriched amount of ligand compounds being capable of binding to a predetermined receptor, comprising the steps:
(a) providing a binding site geometry of said predetermined receptor,
(b) inverting the binding site geometry of said receptor to create a ligand candidate,
(c) generating a computer readable code of said ligand candidate,
(d) providing a description of said ligand candidate in the form of its three-dimensional geometry or/and of its bond distance matrix,
(e) generating a predetermined number of possible ligand conformers of said ligand candidate structure,
(f) providing atomic electrotopological values for the atoms of said ligand candidate structure,
(g) generating atomic types based on said atomic electrotopological values, wherein atoms with atomic electrotopological values between two specified boundaries are assigned to the same atomic type,
(h) generating pharmacophores of the ligand candidate based on said atomic types,
(i) sorting a starting database using said pharmacophores using a similarity index by
(i1) generating a predetermined number of conformers for compounds contained in the database,
(i2) determining pharmacophores for said conformers of said database compounds based on atomic types generated using atomic electrotopological values, and
(i4) comparing the pharmacophores of the ligand structure with the pharmacophores of the database compound,
(j) determining a ranking of the database compounds according to the detected similarities and
(k) obtaining a focussed compound library having an enriched amount of ligand compounds.

## Patentansprüche

1. Verfahren zur Herstellung einer fokussierten Verbindungsbibliothek aus einer Ausgangsverbindungsbibliothek, worin die fokussierte Verbindungsbibliothek eine angereicherte Menge an Ligandenverbindungen enthält, die in der Lage sind an einen vorbestimmten Rezeptor zu binden, umfassend die Schritte:
(a) Bereitstellen wenigstens einer Struktur eines Liganden, eines Ligand-Rezeptor-Komplexes oder einer Ligandenbindungsstellengeometrie für den vorbestimmten Rezeptor,
(b) Erstellen eines Computer-lesbaren Codes der wenigstens einen Struktur,
(c) Bereitstellen einer Beschreibung der wenigstens einen Struktur in Form ihrer dreidimensionalen Geometrie oder/und ihrer Bindungsabstandmatrix,
(d) Bereitstellen atomarer elektrotopologischer Werte für die Atome der wenigstens einen Struktur;
(e) Erstellen von auf den atomaren elektrotopologischen Werten basierenden Atomtypen, worin Atome mit atomaren elektrotopologischen Werten zwischen zwei festgelegten Grenzen dem selben Atomtyp zugerechnet werden,
(f) Erstellen von auf den Atomtypen basierenden Pharmakophoren,
(g) Sortieren einer Ausgangsdatenbank unter Verwendung der Pharmakophore, wobei ein Ähnlichkeitsindex verwendet wird, durch
(g1) Bereitstellen einer Beschreibung der Struktur der in der Datenbank enthaltenen Verbindungen in Form ihrer dreidimensionalen Geometrie oder/und ihrer Bindungsabstandmatrix,
(g2) Bereitstellen atomarer elektrotopologischer Werte für die Atome der wenigstens einen Struktur,
(g3) Erstellen von auf den atomaren elektrotopologischen Werten basierenden Atomtypen,
(g4) Erstellen von auf den Atomtypen basierenden Pharmakophoren, und
(g5) Vergleichen der Pharmakophore der wenigstens einen Ligandenstruktur mit den Pharmakophoren der Verbindungen aus der Datenbank,
(h) Festlegen einer Rangliste der Verbindungen aus der Datenbank gemäß den festgestellten Ähnlichkeiten, und
(i) Erhalten einer fokussierten Verbindungsbibliothek mit einer angereicherten Menge an Ligandenverbindungen.

2. Verfahren gemäß Anspruch 1 zur Herstellung einer fokussierten Verbindungsbibliothek aus einer Ausgangsverbindungsbibliothek, worin die fokussierte Verbindungsbibliothek eine angereicherte Menge an Ligandenverbindungen enthält, die in der Lage sind an einen vorbestimmten Rezeptor zu binden, umfassend die Schritte:
(a) Bereitstellen wenigstens einer Ligandenstruktur für den vorbestimmten Rezeptor,
(b) Erstellen einer vorbestimmten Anzahl an möglichen Ligandenkonformeren der Ligandenstruktur,
(c) Erstellen eines Computer-lesbaren Codes der möglichen Ligandenkonformere,
(d) Bereitstellen einer Beschreibung der möglichen Ligandenkonformere in Form ihrer dreidimensionalen Geometrie oder/und ihrer Bindungsabstandmatrix,
(e) Bereitstellen atomarer elektrotopologischer Werte für die Atome der möglichen Ligandenkonformere,
(f) Erstellen von auf den atomaren elektrotopologischen Werten basierenden Atomtypen, worin Atome mit atomaren elektrotopologischen Werten zwischen zwei festgelegten Grenzen dem selben Atomtyp zugerechnet werden,
(g) Erstellen von auf den Atomtypen basierenden Pharmakophoren,
(h) Sortieren einer Ausgangsdatenbank unter Verwendung der Pharmakophore, wobei ein Ähnlichkeitsindex verwendet wird, durch
(h1) Erstellen einer vorbestimmten Anzahl an Konformeren für die in der Datenbank enthaltenen Verbindungen,
(h2) Bereitstellen einer Beschreibung der Struktur der Konformere in Form ihrer dreidimensionalen Geometrie oder/und ihrer Bindungsabstandmatrix,
(h3) Bereitstellen atomarer elektrotopologischer Werte für die Atome der Konformere,
(h4) Erstellen von auf den atomaren elektrotopologischen Werten basierenden Atomtypen,
(h5) Erstellen von Pharmakophoren für die Konformere für die Verbindungen aus der Datenbank basierend auf den Atomtypen und
(h6) Vergleichen der Pharmakophore der Ligandenstruktur mit den Pharmakophoren der Verbindungen aus der Datenbank und
(i) Festlegen einer Rangliste der Verbindungen aus der Datenbank gemäß den festgestellten Ähnlichkeiten,
(j) Erhalten einer fokussierten Verbindungsbibliothek mit einer angereicherten Menge an Ligandenverbindungen.

3. Verfahren gemäß Anspruch 1, worin der Ähnlichkeitsindex, der zur Sortierung aus der Datenbank verwendet wird, ausgewählt ist aus der Gruppe, die besteht aus Tanimoto-Koeffizient, euklidischer Abstand, Manhatten Abstand und jede beliebige Kombination davon.

4. Verfahren gemäß Anspruch 1, worin Zweipunkt-Pharmakophore (2PP), Dreipunkt-Pharmakophore (3PP) und/oder Vierpunkt-Pharmakophore (4PP) erzeugt werden.

5. Verfahren gemäß Anspruch 2, worin die Konformere durch Kraftfeld-Verfahren oder regelbasierte Verfahren oder Kombinationen davon erzeugt werden.

6. Verfahren gemäß Anspruch 2, worin die vorbestimmte Anzahl der Konformere eine Zahl zwischen 5 und 20 ist.

7. Verfahren gemäß Anspruch 1, worin die Ausgangsverbindungsbibliothek ausgewählt ist aus einer Datenbank bekannter einzelner Verbindungen, einer Datenbank synthetisierter kombinatorischer Bibliotheken und/oder einer Datenbank virtueller kombinatorischer Bibliotheken.

8. Verfahren gemäß Anspruch 1, worin spezifische atomare elektrotopologische Werte, die bei einem Atomtyp und/oder der gesamten Anzahl an Atomtypen berücksichtigt werden, durch Durchmusterung einer Testbibliothek optimiert sind.

9. Verfahren gemäß Anspruch 1 zur Herstellung einer fokussierten Verbindungsbibliothek aus einer Ausgangsverbindungsbibliothek, worin die fokussierte Verbindungsbibliothek eine angereicherte Menge an Ligandenverbindungen enthält, die in der Lage sind an einen vorbestimmten Rezeptor zu binden, umfassend die Schritte:
(a) Bereitstellen einer dreidimensionalen Ligand-Rezeptor-Komplexstruktur für den vorbestimmten Rezeptor,
(b) Ableiten der Ligandenstruktur aus der dreidimensionalen Ligand-Rezeptor-Komplexstruktur,
(c) Erstellen eines Computer-lesbaren Codes der Ligandenstruktur,
(d) Bereitstellen einer Beschreibung der Ligandenstruktur in der Form ihrer dreidimensionalen Geometrie oder/und ihrer Bindungsabstandmatrix,
(e) Bereitstellen atomarer elektrotopologischer Werte für die Atome der Ligandenstruktur,
(f) Erstellen von auf den atomaren elektrotopologischen Werten basierenden Atomtypen, worin Atome mit atomaren elektrotopologischen Werten zwischen zwei festgelegten Grenzen dem selben Atomtyp zugerechnet werden,
(g) Erstellen von auf den Atomtypen und intermolekularen Wechselwirkungen zwischen Ligand und Rezeptor basierenden Pharmakophoren der Ligandenstruktur,
(h) Sortieren einer Ausgangsdatenbank unter Verwendung der Pharmakophore unter Verwendung eines Ähnlichkeitsindex durch
(h1) Erstellen einer vorbestimmten Anzahl an Konformeren für die in der Datenbank enthaltenen Verbindungen,
(h2) Bestimmen der Pharmakophore der Konformere der Verbindungen aus der Datenbank basierend auf Atomtypen, die unter Verwendung atomarer elektrotopologischer Werte erzeugt wurden, und
(h3) Vergleichen der Pharmakophore der Ligandenstruktur mit den Pharmakophoren der Verbindungen aus der Datenbank und
(i) Festlegen einer Rangliste der Verbindungen aus der Datenbank gemäß den festgestellten Ähnlichkeiten und
(j) Erhalten einer fokussierten Verbindungsbibliothek mit einer angereicherten Menge an Ligandenverbindungen.

10. Verfahren gemäß Anspruch 1 zur Herstellung einer fokussierten Verbindungsbibliothek aus einer Ausgangsverbindungsbibliothek, worin die fokussierte Verbindungsbibliothek eine angereicherte Menge an Ligandenverbindungen enthält, die in der Lage sind an einen vorbestimmten Rezeptor zu binden, umfassend die Schritte:
(a) Bereitstellen einer Geometrie einer Bindungsstelle für den vorbestimmten Rezeptor,
(b) Invertieren der Geometrie der Bindungsstelle des Rezeptors, um einen Ligandenkandidaten zu erstellen,
(c) Erstellen eines Computer-lesbaren Codes des Ligandenkandidaten,
(d) Bereitstellen einer Beschreibung des Ligandenkandidaten in der Form seiner dreidimensionalen Geometrie oder/und seiner Bindungsabstandmatrix,
(e) Erstellen einer vorbestimmten Anzahl an möglichen Ligandenkonformeren für die Struktur des Ligandenkandidaten,
(f) Bereitstellen atomarer elektrotopologischer Werte für die Atome der Struktur des Ligandenkandidaten,
(g) Erstellen von auf den atomaren elektrotopologischen Werten basierenden Atomtypen, worin Atome mit atomaren elektrotopologischen Werten zwischen zwei festgelegten Grenzen dem selben Atomtyp zugerechnet werden,
(h) Erstellen von auf den Atomtypen basierenden Pharmakophoren der Ligandenkandidaten,
(i) Sortieren einer Ausgangsdatenbank unter Verwendung der Pharmakophore unter Verwendung eines Ähnlichkeitsindex durch
(i1) Erstellen einer vorbestimmten Anzahl an Konformeren für die in der Datenbank enthaltenen Verbindungen,
(i2) Bestimmen der Pharmakophore der Konformere der Verbindungen aus der Datenbank basierend auf Atomtypen, die unter Verwendung atomarer elektrotopologischer Werte erzeugt wurden, und
(i3) Vergleichen der Pharmakophore der Ligandenstruktur mit den Pharmakophoren der Verbindungen aus der Datenbank,
(j) Festlegen einer Rangliste der Verbindungen aus der Datenbank gemäß den festgestellten Ähnlichkeiten und
(k) Erhalten einer fokussierten Verbindungsbibliothek mit einer angereicherten Menge an Ligandenverbindungen.

## Revendications

1. Procédé pour générer une bibliothèque de composés ciblés à partir d'une bibliothèque de composés de départ dans laquelle ladite bibliothèque de composés ciblés contient une quantité enrichie de composés ligand capables de se lier à un récepteur prédéterminé, comprenant les étapes:
(a) mettre à disposition au moins une structure d'un ligand, un complexe ligand-récepteur ou une géométrie de site de liaison de ligand pour le récepteur prédéterminé,
(b) générer un code lisible par ordinateur de ladite au moins une structure,
(c) mettre en place une description de ladite au moins une structure sous forme de sa géométrie tridimensionnelle et/ou de sa matrice de distance de liaison,
(d) mettre en place des valeurs électrotopologiques atomiques pour les atomes de ladite au moins une structure,
(e) générer les types atomiques basés sur lesdites valeurs électrotopologiques atomiques, dans lesquels les atomes avec les valeurs électrotopologiques atomiques entre deux limites spécifiées sont attribués au même type atomique,
(f) générer les pharmacophores sur la base desdits types atomiques,
(g) trier une base de données de départ en utilisant lesdits pharmacophores, en utilisant un indice de similarité en
(g1) mettant en place une description de la structure des composés contenus dans la base de données sous forme de leur géométrie tridimensionnelle et/ou de leur matrice de distance de liaison,
(g2) fournir les valeurs électrotopologiques atomiques pour les atomes de ladite au moins une structure,
(g3) générer les types atomiques sur la base desdites valeurs électrotopologiques atomiques,
(g4) générer les pharmacophores sur la base desdits types atomiques, et
(g5) comparer les pharmacophores de ladite au moins une structure de ligand avec les pharmacophores des composés de la base de données,
(h) déterminer une classification des composés de la base de données selon les similarités détectées, et
(i) obtenir une bibliothèque de composés ciblés ayant une quantité enrichie de composés ligand.

2. Procédé selon la revendication 1 pour générer une bibliothèque de composés ciblés à partir d'une bibliothèque de composés de départ dans laquelle ladite bibliothèque de composés ciblés contient une quantité enrichie de composés ligand capables de se lier à un récepteur prédéterminé, comprenant les étapes:
(a) mettre en place au moins une structure de ligand pour le récepteur prédéterminé,
(b) générer un nombre prédéterminé de conformères de ligand possibles de ladite structure de ligand,
(c) générer un code lisible par ordinateur des conformères de ligand possibles,
(d) fournir une description des conformères de ligand possibles sous forme de leur géométrie tridimensionnelle et/ou de leur matrice à distance de liaison,
(e) fournir des valeurs électrotopologiques atomiques pour les atomes des conformères de ligand possibles,
(f) générer les types atomiques basés sur lesdites valeurs électrotopologiques atomiques, dans lesquels les atomes avec les valeurs électrotopologiques atomiques entre deux limites spécifiées sont affectés au même type atomique,
(g) générer des pharmacophores sur la base desdits types atomiques,
(h) trier une base de données de départ en utilisant lesdits pharmacophores, en utilisant un indice de similarité en
(h1) générant un nombre prédéterminé de conformères pour les composés contenus dans la base de données,
(h2) mettre en place une description de la structure desdits conformères sous forme de leur géométrie tridimensionnelle et/ou de leur matrice de distance de liaison,
(h3) mettre en place des valeurs électrotopologiques atomiques pour les atomes desdits conformères,
(h4) générer les types atomiques sur la base desdites valeurs électrotopologiques atomiques,
(h5) générer les pharmacophores pour lesdits conformères desdits composés de la base de données en fonction desdits types atomiques et
(h6) comparer les pharmacophores de la structure de ligand avec les pharmacophores des composés de la base de données et
(i) déterminer une classification des composés de la base de données selon les similarités détectées,
(j) obtenir une bibliothèque de composés ciblés ayant une quantité enrichie de composés ligand.

3. Procédé selon la revendication 1, dans lequel l'indice de similitude utilisé pour trier la base de données est choisi dans le groupe consistant en le coefficient Tanimoto, la distance Euclidienne, la distance Manhattan et toute combinaison de celles-ci.

4. Procédé selon la revendication 1, dans lequel les pharmacophores à deux points (2PP), les pharmacophores à trois points (3PP) et/ou les pharmacophores à quatre points (4PP) sont générés.

5. Procédé selon la revendication 2, dans lequel les conformères sont générés par champs de force ou par des procédés à base de règles ou de leurs combinaisons.

6. Procédé selon la revendication 2, dans lequel le nombre prédéterminé de conformères est un nombre entre 5 et 20.

7. Procédé selon la revendication 1, dans lequel la bibliothèque de composés de départ est choisie à partir d'une base de données de composés individuels connus, une base de données de bibliothèques combinatoires synthétisées et/ou d'une base de données de bibliothèques combinatoires virtuelles.

8. Procédé selon la revendication 1, dans lequel les valeurs électrotopologiques atomiques spécifiques qui sont incorporées dans un type atomique et/ou le nombre total de types atomiques sont optimisés par criblage d'une bibliothèque test.

9. Procédé selon la revendication 1 pour générer une bibliothèque de composés ciblés à partir d'une bibliothèque de composés de départ, dans lequel ladite bibliothèque de composés ciblés contient une quantité enrichie de composés ligand capables de se lier à un récepteur prédéterminé, comprenant les étapes:
(a) mettre en place une structure de complexe-récepteur-ligand tridimensionnelle pour le récepteur prédéterminé,
(b) dériver la structure de ligand à partir de la structure complexe-récepteur-ligand tridimensionnelle,
(c) générer un code lisible par ordinateur de ladite structure de ligand,
(d) fournir une description de ladite structure de ligand sous forme de sa géométrie tridimensionnelle et/ou de sa matrice de distance de liaison,
(e) fournir des valeurs électrotopologiques atomiques pour les atomes de ladite structure de ligand,
(f) générer les types atomiques en fonction desdites valeurs électrotopologiques atomiques, dans lesquels les atomes avec les valeurs électrotopologiques atomiques entre deux limites spécifiées sont affectés au même type atomique,
(g) générer les pharmacophores de la structure de ligand en fonction desdits types atomiques et des interactions intermoléculaires entre récepteur et ligand,
(h) trier une base de données de départ en utilisant les pharmacophores, en utilisant un indice de similarité en
(h1) générant un nombre prédéterminé de conformères pour les composés contenus dans la base de données,
(h2) déterminant les pharmacophores pour lesdits conformères desdits composés de la base de données en fonction des types atomiques générés en utilisant des valeurs électrotopologiques atomiques, et
(h3) comparant les pharmacophores de la structure de ligand avec les pharmacophores des composés de la base de données et
(i) déterminer une classification des composés de la base de données selon les similarités détectées et
(j) obtenir une bibliothèque de composés ciblés ayant une quantité enrichie de composés ligand.

10. Procédé selon la revendication 1 pour générer une bibliothèque de composés ciblés à partir d'une bibliothèque de composés de départ dans lesquels ladite bibliothèque de composés ciblés contient une quantité enrichie de composés ligand capables de se lier à un récepteur prédéterminé, comprenant les étapes :
(a) mise en place d'une géométrie de site de liaison dudit récepteur prédéterminé,
(b) inverser la géométrie du site de liaison dudit récepteur pour créer un candidat ligand,
(c) générer un code lisible par ordinateur dudit candidat ligand,
(d) fournir une description dudit candidat de ligand sous forme de sa géométrie tridimensionnelle et/ou de sa matrice de distance de liaison,
(e) générer un nombre déterminé de conformères de ligand possible de ladite structure de candidat ligand,
(f) fournir les valeurs électrotopologiques atomiques pour les atomes de ladite structure du candidat ligand,
(g) générer les types atomiques sur la base desdites valeurs électrotopologiques atomiques, dans lequel les atomes avec les valeurs électrotopologiques atomiques entre deux limites spécifiées sont affectés au même type atomique,
(h) générer les pharmacophores du candidat ligand sur la base desdits types atomiques,
(i) trier une base de données de départ en utilisant lesdits pharmacophores, en utilisant l'indice de similarité en
(i1) générant un nombre prédéterminé de conformères pour les composés contenus dans la base de données,
(i2) déterminant les pharmacophores pour lesdits conformères desdits composés de la base de données en fonction des types atomiques générés en utilisant des valeurs électrotopologiques atomiques, et
(i4) comparant les pharmacophores de la structure de ligand avec les pharmacophores du composé de la base de données,
(j) déterminer une classification des composés de la base de données selon les similitudes détectées et
(k) obtenir une bibliothèque de composés ciblés ayant une quantité enrichie de composés ligand.
